(19) Europäisches Patentamt
European Patent Office
Office européen des brevets

(11) Veröffentlichungsnummer: **0 116 288**
**A2**

(12) # EUROPÄISCHE PATENTANMELDUNG

(21) Anmeldenummer: **84100240.5**

(22) Anmeldetag: **11.01.84**

(51) Int. Cl.$^3$: **C 08 G 69/44**
A 61 L 2/26, C 08 J 5/18

(30) Priorität: **13.01.83 DE 3300944**

(43) Veröffentlichungstag der Anmeldung:
**22.08.84 Patentblatt 84/34**

(84) Benannte Vertragsstaaten:
**AT BE CH DE FR GB IT LI LU NL SE**

(71) Anmelder: **Intermedicat GmbH**
**Gerliswilstrasse 45**
**CH-6020 Emmenbrücke(CH)**

(72) Erfinder: **Brethauer, Ulrich**
**Rhönstrasse 2**
**D-3501 Körle(DE)**

(74) Vertreter: **von Kreisler, Alek, Dipl.-Chem. et al,**
**Deichmannhaus am Hauptbahnhof**
**D-5000 Köln 1(DE)**

(54) **Sterilisierfähige Folienmaterialien für Primärverpackungen von wässrigen Lösungen im medizinischen Bereich.**

(57) Die Erfindung betrifft sterilisierfähige Folienmaterialien für Primärverpackungen von wässrigen Lösungen im medizinischen Bereich, die dadurch gekennzeichnet sind, daß sie aus einem thermoplastischen Blockcopolymeren der allgemeinen Formel (I)

$$\left[ -\underset{\underset{O}{\|}}{C} - A - \underset{\underset{O}{\|}}{C} - O - B - O - \right]_n$$

bestehen, in der A lineare gesättigte aliphatische Polyamidsequenz, gebildet aus einem Lactam oder einer Aminosäure mit einer Kohlenwasserstoffkette von 4 bis 14 C-Atomen oder aus einer aliphatischen, 6 bis 12 C-Atome enthaltenden Dicarbonsäure und einem 6 - 9 C-Atome enthaltenden Diamin in Gegenwart einer die Kettenlänge begrenzenden aliphatischen Dicarbonsäure mit 4 bis 20 C-Atomen, bedeutet, wobei das Polyamid en mittleres Molekulargewicht zwischen 300 und 15 000 hat; B eine Polyoxyalkylensequenz, gebildet aus linearen oder verzweigten aliphatischen Polyoxyalkylenglykolen, ihren Mischungen oder Copolyetherderivaten bedeutet, wobei die Polyoxyalkylenglykole ein mittleres Molekulargewicht 6000 haben; und n die Zahl der sich wiederholenden Polymereinheiten ist, die so groß ist, daß das Polyether-Ester-Amid-Blockcopolymer eine Grenzviskosität von 0,8 bis 2,05 hat.

Die Erfindung betrifft außerdem Verbundfolien, in denen je eine Schicht des Folienmaterials des Blockcopolymeren der allgemeinen Formel (I) eine Schicht eines anderen thermoplastischen Materials auf beiden Seiten einschließt, sowie die Verwendung der genannten Folienmaterialien zur Herstellung von Primärverpackungen für wässrige Lösungen im medizinischen Bereich.

EP 0 116 288 A2

Croydon Printing Company Ltd.

# Sterilisierfähige Folienmaterialien für Primärverpackungen von wässrigen Lösungen im medizinischen Bereich

Die Erfindung betrifft sterilisierfähige Folienmaterialien und deren Verwendung für Primärverpackungen von wässrigen Lösungen im medizinischen Bereich.

Infusions- und Übertragungslösungen werden herkömmlich in Glasflaschen, Kunststoffbehältern und Beutelbehältnissen angeboten. Aufgrund der durch das Gerätematerial Glas bedingten Nachteile (energiebedingt hohe Herstellungskosten, relativ hohes Behältergewicht, fehlende Fallfestigkeit und problematische Entsorgung der entleerten Glasflaschen) ging man mehr und mehr dazu über, zur Lagerung bzw. Übertragung wässriger Lösungen im medizinischen Bereich Behältnisse aus Kunststoff zu verwenden. Dabei bieten Beutelsysteme aus weichmacherhaltigen Polyvinylchlorid-Folien ganz entscheidende Vorteile, die ihren Einsatz im Markt bislang konkurrenzlos gestalteten: die Herstellung von weichmacherhaltigen PVC-Beuteln kann mit hoher Frequenz erfolgen, ohne daß Probleme beim Einschweißen von Zu- und Ableitungssystemen auftreten, die ebenfalls aus PVC hergestellt sind, wie z.B. Füllrohre, Einstechstutzen mit Membran und Injektionskanäle mit Kautschukmembranen. Die so erhaltenen Beutel weisen eine hohe Flexibilität auf, sind weitgehend transparent und reißen selbst dann nicht auf, wenn sie in gefülltem Zustand zu Boden fallen.

Gerade im medizinischen Bereich ist jedoch die Verwendung von mit Weichmacher plastifiziertem Polyvinylchlorid für Folienmaterialien bzw. genannte Beutelsysteme nicht unumstritten. Es muß nämlich damit gerechnet werden, daß der Beutelinhalt, insbesondere Fett- und Blutserum-haltige Lösungen, die Weichmacher, in der Regel Di-2-ethylhexylphthalat, aus dem Folienmaterial herauslösen und so mit bestimmungsfremden Verbindungen belastet werden. Zudem treten die Wirkstoffe spezieller Lösungen z.B. durch Adsorption mit Weich-PVC in Wechselwirkung.

Üblicherweise werden Beutel aus Weich-PVC im Umbeutel bei 110°C/40 min. oder bei 121°C/20 min. dampfsterilisiert. Unmittelbar nach der Dampfsterilisation ist das Beutelmaterial milchig, so daß der Inhalt nur unzureichend beurteilt werden kann. Erst im Verlauf längerer Lagerung wird die Transparenz besser.

Ein dem zunehmenden Umweltbewußtsein Rechnung tragender Einwand ist, daß bei der Entsorgung der leeren Weich-PVC-Beutel in Müllverbrennungsanlagen agressive Chlorwasserstoffdämpfe freigesetzt werden und aus diesem Grunde ihre Verwendung eingeschränkt werden muss.

Der vorliegenden Erfindung liegt die Aufgabe zugrunde, ein Alternativmaterial zum marktüblichen, mit Weichmachern plastifizierten Polyvinylchlorid für Infusionslösungs-Beutel zu entwickeln. Es soll somit ein Material gefunden werden, das die geschilderten Nachteile von Weich-PVC nicht mehr aufweist und gleichzeitig dessen Vorteile in ein neues Konzept einbringt.

Demgemäß betrifft die Erfindung sterilisierfähige Folienmaterialien für Primärverpackungen von wässrigen Lösungen im medizinischen Bereich, die aus einem thermoplastischen Blockcopolymeren, nämlich einem Polyether-Ester-Amid-Blockcopolymeren der allgemeinen Formel (I)

$$\left[ -\underset{\overset{\|}{O}}{C} - A - \underset{\overset{\|}{O}}{C} - O - B - O - \right]_n$$

bestehen, in der A eine lineare gesättigte aliphatische Polyamidsequenz, gebildet aus einem Lactam oder einer Aminosäure mit einer Kohlenwasserstoffkette von 4 bis 14 C-Atomen oder aus einer aliphatischen, 6 bis 12 C-Atome enthaltenden Dicarbonsäure und einem 6 - 9 C-Atome enthaltenden Diamin in Gegenwart einer die Kettenlänge begrenzenden aliphatischen Dicarbonsäure mit 4 bis 20 C-Atomen, bedeutet, wobei das Polyamid ein mittleres Molekulargewicht zwischen 300 und 15 000 hat; B eine Polyoxyalkylensequenz, gebildet aus linearen oder verzweigten aliphatischen Polyoxyalkylenglykolen, ihren Mischungen oder Copolyetherderivaten bedeutet, wobei die Polyoxyalkylenglykole ein mittleres Molekulargewicht 6000 haben; und n die Zahl der sich wiederholenden Polymereinheiten ist, die so groß ist, daß das Polyether-Ester-Amid-Blockcopolymer eine Grenzviskosität von 0,8 bis 2,05 hat.

Das genannte Polyether-Ester-Amid-Blockcopolymere ist Gegenstand der US-PS 4 331 786.

Das Material der angegebenen allgemeinen Formel (I) zeigt in seiner chemischen Struktur eine lineare regelmässige Kette von steifen Polyamid- und flexiblen Polyether-Segmenten.

Die erfindungsgemässen Folien enthalten keine Weichmacher im Sinne einer bei Weich-PVC üblichen äußeren Weichmachung.

Die Folien aus Polyetherblockamid können als Flachfolien oder als Blasfolien hergestellt werden. Dies geschieht dadurch, daß das Polyetherblockamid-Granulat in einen Extruder eingefüllt und mittels bekannter Methoden durch geeignete Düsen aus diesem ausgetragen wird. Sofort hinter der Düse wird das Folienmaterial im noch plastischen Zustand durch Luft auf ein Mehrfaches des Austrittsdurchmessers aufgeblasen und dann zwischen einem Walzenpaar flachgequetscht oder flachgedreht. Zur Herstellung von Flachfolien schneidet man die beiden seitlichen Faltkanten ab; zur Herstellung von Blasfolien kann das so hergestellte Schlauchband direkt weiterverarbeitet werden.

Aus Gründen der Hygiene und der verunreinigungsfreien Weiterverarbeitung sind Blasfolien für die Verwendung im medizinischen Gebrauch vorzuziehen.

Der Aufblasprozess ermöglicht es, sehr weite Schläuche mit dünnen Wanddicken zu erzeugen, ohne daß die Düse ebenso groß sein und einen besonders engen und damit empfindlichen Spalt haben müsste wie der spätere Folienschlauch. So kann nach dem Blasextrusionsverfahren eine Mono-Schlauchfolie mit einer Wanddicke von 100 - 300 µm, vorzugsweise 150 - 200 µm, hergestellt werden.

Folien der genannten Materialien und Wanddicken lassen sich außerordentlich gut mittels Wärmekontakt oder Wärmeimpuls zu Beuteln verschweißen, vorzugsweise im Hochfrequenzverfahren. Ein Beutel aus dieser Folie mit einer wässrigen Infusionslösung als Inhalt läßt sich problemlos im Gegendruck-Autoklaven bei 121°C und einer

Belastungszeit von 20 Minuten ohne Umbeutel sterilisieren, ohne daß er thermisch deformiert wird. Die Transparenz des Folienmaterials verschlechtert sich dabei nicht.

Der sterilisierte Beutel zeigt hervorragende mechanische Eigenschaften. Bei einem Test der Fallfestigkeit nach DIN 58 363, Teil 15, Punkt 4.1.4 wurde eine sehr gute Fallfestigkeit gefunden.

Neben der Herstellung von Monofolien, wie oben beschrieben, ist auch die Herstellung von Mehrschichtfolien möglich. Dazu eignen sich zahlreiche andere thermoplastische, für Folienherstellung geeignete Werkstoffe wie z.B. Polypropylen, High-Density-Polyethylen, Polyvinylalkohol, Polyethylenterephthalat, Polybutylenterephthalat, Linear-Low-Density-Polyethylen-Ionomer, Polyamid, elastomere Copolymere aus Ethylen und Vinylacetat, Celluloseacetat oder Polyvinylidenchlorid. Zweck derartiger Mehrschichtenfolien, die aus einem Verbund aus Polyetherblockamid mit den genannten thermoplastischen Materialien bestehen, ist die Herstellung von Folienbeuteln mit Gas- und Wasserdampfsperren.

So konnte beispielsweise zur Verringerung der Sauerstoff- und Wasserdampfdurchlässigkeit eine Folie im Coextrusions-Blasverfahren mit drei Schichten aus Polyetherblockamid/Polyvinylidendichlorid/Polyetherblockamid hergestellt werden.

Die Erfindung wird durch die nachfolgenden Beispiele erläutert.

Beispiel 1

Auf einer Folienblasanlage wurde mit dem beschriebenen Copolyether-Ester-Amid eine Mono-Schlauchfolie extrudiert.

Der Extruder hatte eine Schnecke mit 60 mm Durchmesser und eine Länge von 25 d. Damit verbunden war eine Ringdüse mit 200 mm Durchmesser.

Extrusionsbedingungen:

| | |
|---|---|
| Schneckenumdrehung: | 65 UpM |
| Leistungsaufnahme des Extruders: | 75 A |
| Zylindertemperatur: | 200 - 225°C |
| Temperatur am Zwischenstück und Kopf: | 225°C |
| Massentemperatur: | 235°C |
| Druck vor dem Sieb: | 115 kp/cm² |
| Zuggeschwindigkeit der Folie: | 10 m/min. |
| Folienbreite: | 685 mm |
| Foliendicke: | 140 - 160 µm |

Die Folie wurde anschließend im Hochfrequenzschweißverfahren zu 500 ml-Beutelbehältnissen verarbeitet. Gleichzeitig wurden die Beutelzu- und -ableitungssysteme eingeschweißt. Die Zu- und Ableitungssysteme waren wie die Folie ebenfalls aus Copolyether-Ester-Amid und waren im Spritzgußverfahren und im Schlauchextrusionsverfahren hergestellt worden.

Die so erhaltenen Beutel wurden mit einer wässrigen Infusionslösung (0,9 %ige physiologische Kochsalzlösung) befüllt und ohne Umbeutel im Gegendruckautoklaven bei 121°C/20 min. dampfsterilisiert.

Anschließend wurden die sterilisierten Beutel gegen nichtsterilisierte Beutel beurteilt.

Die Transparenz war ebenso gut wie vor der Sterilisation.
Deformierungserscheinungen durch die Temperaturbelastung konnten am Behältermaterial nicht festgestellt werden.

Nach DIN 58 363, Teil 15, Punkt 4.1.4 wurde mit 5 Beuteln ein Falltest gemacht. Alle 5 Beutel hielten diesen Test aus.

Allerdings waren die Sperreigenschaften gegenüber Wasserdampf und Sauerstoff noch nicht zufriedenstellend. Weitere Beutel wurden daraufhin in einen dampfsterilisierfähigen, transparenten Umbeutel eingeschweißt. Als Umbeutelmaterial kamen handelsübliche Folien wie z.B. Polyamid/Polyethylen (50 µm/75 µm) und Polyamid/-Polypropylen (70 µm/75 µm) zum Einsatz. Dabei bietet die Polyamid-Schicht eine ausreichende Sauerstoffsperreigenschaft und die Polyolefin-Schicht eine ausreichende Wasserdampfsperreigenschaft. Es wurde im Versuch darauf geachtet, daß die Umbeutel möglichst dicht an dem Lösungsbeutel anlagen. Mit Hilfe von Vakuum konnte dies erreicht werden.

Beispiel 2

Eine weitere Ausführungsform zur Verbesserung der Wasserdampf- und Sauerstoffsperreigenschaften an der erfindungsgemäßen Folie ist die direkte Kombination mit einer oder mehreren Sperrschichten. Im Koextrusionsverfahren wurde eine 3-schichtige Schlauchfolie geblasen. Geplant war eine Folie mit folgendem Aufbau:

Copolyether-Ester-Amid/Polyvinylidenschlorid/Copolyether-Ester-Amid

Die Maschinenbedingungen bei der Extrusion wurden wie in Beispiel 1 gewählt, jedoch folgende Änderungen zusätzlich eingeführt:

3 Materialien wurden mit 3 Extrudern in einer Ringdüse zusammengeführt.
Die Extruder 1 und 2 förderten Copolyether-Ester-Amid und der 3. Extruder Polyvinylidenchlorid in die Ringdüse.

Erhalten wurde eine transparente Schlauchfolie mit folgenden Schichtdicken:

| | |
|---|---|
| Copolyether-Ester-Amid | 90 - 100 µm |
| Polyvinylidenchlorid-Innenschicht | 15 - 20 µm |
| Copolyether-Ester-Amid | 90 - 100 µm |

Aus dieser Folie wurden Beutel mit Anschlußsystemen im Hochfrequenzschweißverfahren hergestellt und diese gemäß den Prüfungen in Beispiel 1 beurteilt.

Die gefüllten Beutel zeigten nach der Dampfsterilisation bei 121°C/20 min. gleich gute Ergebnisse. Im Gegensatz zur Mono-Schlauchfolie wurden zusätzlich gute Gas- und Wasserdampfsperreigenschaften gemessen.

Patentansprüche

1. Sterilisierfähige Folienmaterialien für Primärverpackungen von wässrigen Lösungen im medizinischen Bereich, dadurch gekennzeichnet, daß sie aus einem thermoplastischen Blockcopolymeren der allgemeinen Formel (I)

$$\left[ - \underset{\underset{O}{\parallel}}{C} - A - \underset{\underset{O}{\parallel}}{C} - O - B - O - \right]_n$$

bestehen, in der A eine lineare gesättigte aliphatische Polyamidsequenz, gebildet aus einem Lactam oder einer Aminosäure mit einer Kohlenwasserstoffkette von 4 bis 14 C-Atomen oder aus einer aliphatischen, 6 bis 12 C-Atome enthaltenden Dicarbonsäure und einem 6 - 9 C-Atome enthaltenden Diamin in Gegenwart einer die Kettenlänge begrenzenden aliphatischen Dicarbonsäure mit 4 bis 20 C-Atomen, bedeutet, wobei das Polyamid ein mittleres Molekulargewicht zwischen 300 und 15 000 hat; B eine Polyoxyalkylensequenz, gebildet aus linearen oder verzweigten aliphatischen Polyoxyalkylenglykolen, ihren Mischungen oder Copolyetherderivaten bedeutet, wobei die Polyoxyalkylenglykole ein mittleres Molekulargewicht 6000 haben; und n die Zahl der sich wiederholenden Polymereinheiten ist, die so groß ist, daß das Polyether-Ester-Amid-Blockcopolymer eine Grenzviskosität von 0,8 bis 2,05 hat.

2. Folienmaterialien nach Anspruch 1, dadurch gekennzeichnet, daß sie eine Dicke von 100 - 300 µm, bevorzugt 150 - 200 µm aufweisen.

3. Folienmaterialien nach Ansprüchen 1 und 2, dadurch gekennzeichnet, daß sie ohne thermische Deformation und ohne Verlust ihrer Transparenz bei 121°C 20 Minuten ohne Umbeutel sterilisiert werden können.

4. Sterilisierfähige Folienmaterialien, dadurch gekennzeichnet, daß sie als Verbundfolien aus mehreren Schichten ausgebildet sind, wobei je eine Schicht der Folienmaterialien nach Anspruch 1 - 3 eine Schicht eines anderen thermoplastischen Materials auf beiden Seiten einschließt.

5. Folienmaterialien nach Anspruch 4, dadurch gekennzeichnet, daß als thermoplastische Materialien Polypropylen, High-Density-Polyethylen, Polyvinylalkohol, Polyethylenterephthalat, Polybutylenterephthalat, Linear-Low-Density-Polyethylen, Ionomer, Polyamid, elastomere Copolymere aus Ethylen und Vinylacetat, Celluloseacetat oder Polyvinylidenchlorid verwendet werden.

6. Verwendung der Folienmaterialien nach Ansprüchen 1 bis 5 zur Herstellung von Primärverpackungen für wässrige Lösungen im medizinischen Bereich.